# EUROPEAN PATENT SPECIFICATION

(11) **EP 2 528 503 B1**
(45) Date of publication and mention of the grant of the patent: **16.07.2014**
(21) Application number: 11704317.4
(22) Date of filing: 19.01.2011
(51) Int. Cl.: A61B 5/06, A61N 1/05, A61N 1/36

(54) **A system and a method for determining an orientation of a biomedical stimulation device**
System und Verfahren zur Bestimmung einer Ausrichtung eines biomedizinischen Geräts
Système et procédé pour déterminer une orientation d'un stimulateur biomédical

(30) Priority: 26.01.2010 EP 10151691
(43) Date of publication of application: 05.12.2012
(73) Proprietor: Sapiens Steering Brain Stimulation B.V., 5656 AE Eindhoven (NL)
(72) Inventor: VAN KAAM, Patrick Wilhelmus, NL-5656 AE Eindhoven (NL); KLEIBEUKER, Johan Gerard, NL-5656 AE Eindhoven (NL); MARTENS, Hubert Cécile François, NL-5656 AE Eindhoven (NL); TOADER, Emil-Codrut, NL-5656 AE Eindhoven (NL)
(74) Representative: Prinz & Partner
(86) International application number: PCT/IB2011/050245
(87) International publication number: WO 2011/092613

(56) References cited:
- WO-A2-2009/044271
- US-A- 5 211 165
- US-A1- 2003 117 270
- US-A1- 2009 253 985

## Description

### FIELD OF THE INVENTION

The invention relates to a system for determination of an orientation of a biomedical stimulation device, for example a deep brain stimulation device, in respect to an object, for example a human being.

The invention also relates to a method for determination of an orientation of a biomedical stimulation device in respect to an object.

### BACKGROUND OF THE INVENTION

Biomedical stimulation devices for stimulating tissue of an object, for example a human being or an animal, are being used for treatment of a wide variety of medical conditions. Using such biomedical stimulation devices, electrical stimulation pulses produced by a pulse generator are conveyed to a desired stimulation site of the object. In order to achieve the desired effects from the delivery of stimulating pulses, it is important that stimulation-delivery elements of the device are properly positioned and oriented so that optimal stimulating energy is applied to the desired stimulation site. While this is true for many different kinds of stimulation therapies, device positioning and orienting is especially critical in the area of neurological stimulation.

When inserting the biomedical stimulation device in an object's tissue it must be possible to accurately determine an orientation, i.e. an angular position, of the device with respect to an object's anatomy. For example, from an angular point of view, a cylindrical biomedical stimulation device that comprises stimulator elements that allow the delivery of stimulation in selective directions can be inserted in the tissue in an infinite number of ways. Thus, a system for orientation detection of the biomedical stimulation device is required.

Known systems are using high-resolution Computed Tomography (CT) imaging devices for determining the position and orientation of the biomedical stimulation device inserted in the object's tissue. In this case the biomedical stimulation device must be of an asymmetrical shape in order that the orientation of the device can be determined on basis of an acquired CT image. A drawback of the known system is that it is relatively complex and expensive. The Computed Tomography (CT) imaging devices are relatively expensive and also manufacturing of biomedical stimulation devices having the asymmetrical shape is relatively expensive. The document US 5 211 165 A discloses a system with transmit coils, receive coils and a tracking and display unit configured to calculate the position and orientation of an invasive device.

### SUMMARY OF THE INVENTION

It is an object of the present invention to provide a system of a reasonable price that is able to accurately determine an angular position of a biomedical stimulation device with respect to an object's anatomy, in particular when the biomedical stimulation device is implanted in an object's tissue. This object is achieved with the system according to the invention as defined in Claim 1. The system for determination of an orientation of a first biomedical stimulation device in respect to an object comprises the first biomedical stimulation device comprising at least two stimulator elements and a generator arranged for feeding the stimulator elements with time-varying electric signals. The time-varying electric signals differ from each other by a predetermined phase. The system further comprises a sensor device with a known relative position with respect to the first biomedical stimulation device, arranged for sensing a sensing signal from the stimulator elements. The sensing signal is originated from the time-varying electric signals from the stimulator elements. The system further comprises a processor device arranged for determining the orientation of the first biomedical stimulation device based on a sensed phase of the sensing signal, phases of the time-varying signals and the known relative position. The angular position of the biomedical stimulation device with respect to the object's anatomy can be obtained using the above described system. Since the functioning of the system does not require relatively expensive Computed Tomography (CT) imaging devices or a biomedical stimulation device having an asymmetrical shape, such a system is relatively cheap, i.e. the system does not have the problem of the prior art.

The system can comprise multiple sensor devices, each of them having a different relative position with respect to the first biomedical stimulation device. Having multiple sensor devices further improves the accuracy of determination of the angular position of the biomedical stimulation device with respect to the object's anatomy.

An embodiment of the system according to the invention has the feature that the first biomedical stimulation device is implantable in the object. This means that the orientation of the device can be determined by a user who is not able to see the device since the device is implanted.

An embodiment of the system according to the invention has the feature that the first biomedical stimulation device is a first deep brain stimulation device.

An embodiment of the system according to the invention has the feature that the sensor device is implantable in the object.

An embodiment of the system according to the invention has the feature that the sensor device is a second biomedical stimulation device.

An embodiment of the system according to the invention has the feature that the second biomedical stimulation device is a second deep brain stimulation (DBS) device.

An embodiment of the system according to the invention has the feature that the time-varying electric signals are sinusoidal patterns.

In another aspect of the invention the said object of the present invention is achieved with the method as defined in claim 8. The method for determination of an orientation of a first biomedical stimulation device in respect to an object, wherein the first biomedical stimulation device comprises stimulator elements, comprises the following steps:
- feeding the stimulator elements by a generator with time-varying electric signals, wherein the time-varying electric signals differ from each other by a predetermined phase,
- sensing a sensing signal from the stimulator elements by a sensor device with a known relative position with respect to the first biomedical stimulation device, wherein the sensing signal is originated from the time-varying electric signals from the stimulator elements, and
- determining the orientation of the first biomedical stimulation device based on a sensed phase of the sensing signal, phases of the time-varying signals and the known relative position by a processor device.

Similarly to the system according to the invention, the method does not involve usage of relatively expensive Computed Tomography (CT) imaging devices or usage of a biomedical stimulation device having an asymmetrical shape. Thus, the implementation of the method is relatively cheap.

### BRIEF DESCRIPTION OF THE DRAWINGS

In the following, the invention and further aspects will be described, by way of example, and explained hereinafter, using the following figures:
Fig. 1 schematically shows a first exemplary embodiment of the system according to the invention;
Figs. 2A, 2B, 2C and 2D schematically show a second exemplary embodiment of the system according to the invention wherein both a first biomedical stimulation device and a sensor device are deep brain stimulation (DBS) devices.

### DETAILED DESCRIPTION OF THE EMBODIMENTS

In the following description of the preferred embodiments, reference is made to the accompanying drawings which form a part thereof. Specific embodiments, in which the invention may be practiced, are shown in the following description by a way of illustration. It is also understood that other embodiments may be utilized and structural changes may be made without departing from the scope of the present invention. It is noted that the same reference signs will be used for indicating the same or similar parts in the several embodiments.

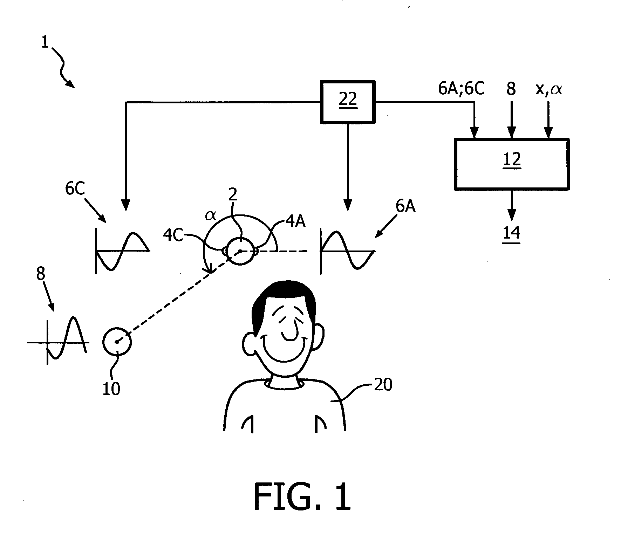

A first embodiment of the invention is shown in Fig. 1. A system 1 for determination of an orientation 14 of a first biomedical stimulation device 2 in respect to an object 20, in particular a human being, comprises the first biomedical stimulation device 2, a sensor device 10, a generator 22 and a processor device 12. The first biomedical stimulation device 2 can be for example a first deep brain stimulation (DBS) device. The generator 22 feeds two stimulator elements 4A;4C of the first biomedical stimulation device 2 with time-varying electric signals 6A;6C, preferably corresponding to a dipole. The time-varying electric signals 6A;6C can form for example sinusoidal patterns. The time-varying electric signals 6A;6C differ from each other by a predetermined phase, for example the predetermined phase of 180 degrees. A relative position X;α of the sensor device 10 in respect to the first biomedical stimulation device 2 is known. The sensor devices 10 senses a sensing signal 8 from the stimulator elements 4A;4C. The sensing signal 8 is originated from the time-varying electric signals 6A;6C from the stimulator elements 4A;4C. Phases of the time-varying electric signals 6A;6C, a sensing phase of the sensing signal 8 and the relative position X;α are provided to the processor device 12. Having these data, the processor device 12 is accurately determining the orientation 14 of the first biomedical stimulation device 2.

Thus, a user of the system 1 is able to determine the orientation of the first biomedical stimulation device 2 in respect to the object 20 also when the user is not able to see the first biomedical stimulation device 2. For example the user, a doctor, can be located in a first room and the object 20, a patient, can be located in a different room. Although the doctor is not able to see the first biomedical stimulation device 2, he is able to determine the device's orientation using the system. The same is true when the first biomedical stimulation device 2 is implanted in the object 20.

Also the sensor device 10 can be implanted in the object 20. Also the sensor device 10 can be a biomedical stimulation device, i.e. a second biomedical stimulation device. Such device, as known in the art, is not only able to provide the stimulation but it is also able to sense electrical signals. Similar to the first biomedical stimulation device 2, the second biomedical stimulation device 10 can be a second deep brain stimulation (DBS) device.

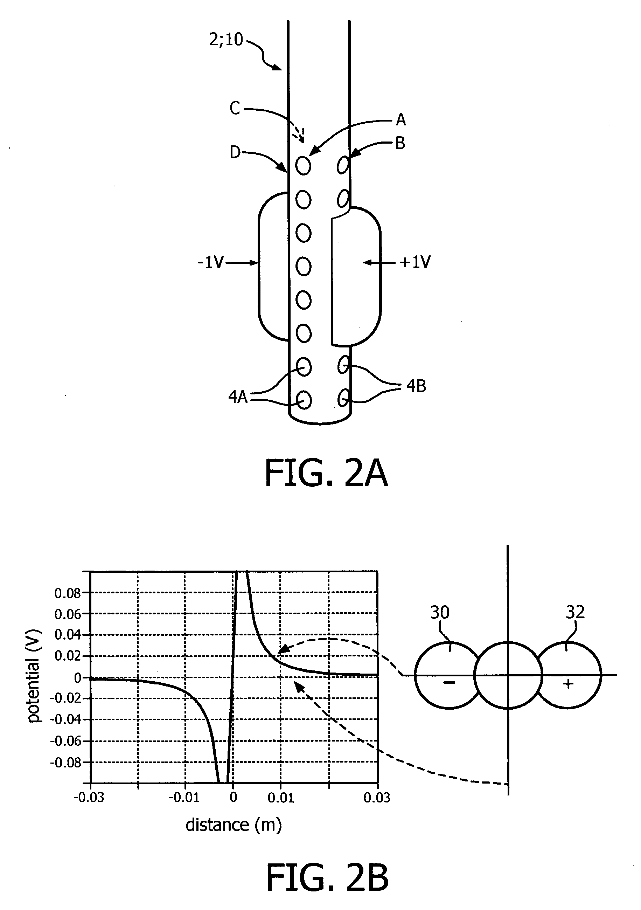

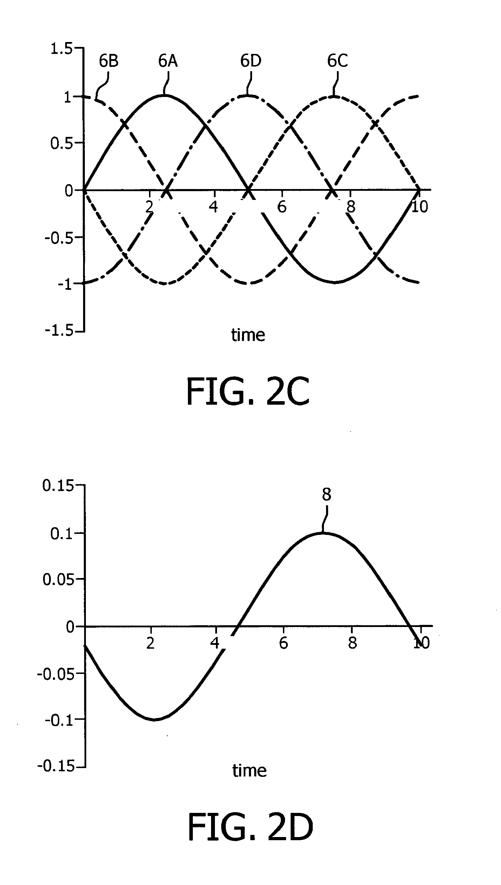

A second embodiment of the invention is shown in Figs. 2A, 2B, 2C and 2D. Both, a first biomedical stimulation device 2 and a sensor device are deep brain stimulation (DBS) devices, i.e. a first deep brain stimulation (DBS) device and a second deep brain stimulation (DBS) device. Such a deep brain stimulation device is shown in Fig. 2A.

The DBS device comprises in the example shown in Fig. 2A four columns A, B, C and D. Each of the columns comprises at least one electrode. In the example according to Fig. 2A each of the columns A, B, C and D comprises eight electrodes. Two DBS devices 2;10 are implanted in a patient 10, for example the first DBS device 2 in a first brain hemisphere and the second DBS device 10 in a second brain hemisphere of the patient.

The DBS devices 2;10 have the ability to deliver electrical signals to the brain tissue and they have the ability to measure electrical signals from the brain tissue. As known in the art, this can be achieved with dedicated electrode arrays for stimulation and sensing, or with multi-functional electrode-arrays capable of providing stimulation or picking up electrical signals. In use for determining the orientation of one of the DBS devices, for example the first DBS device, this device is used for providing electrical fields and the other DBS device, for example the second DBS device, is used for sensing. A dipole shaped field as illustrated in Fig. 2B is created around the first DBS device by applying the appropriate electrical signals on the electrodes of column B and column D. The dipole is oriented with a known angular orientation with respect to the first DBS device. In the particular example this is achieved by providing a negative signal to four electrodes in a column D, which electrodes are not visible in Fig. 2A, and a positive signal to four electrodes in a column B. As a result, a dipole shaped field results around the first DBS device, with negative potentials in a direction of the D column, i.e. the left side in Fig. 2B, and positive potentials in a direction of the B column, i.e. the right side in Fig. 2B and a substantial zero volt plane in the middle. This is illustrated by the voltage cross-section plots shown in Fig. 2B. A negative potential field 30 results in the tissue adjacent to the column D of the first DBS device 2, a positive potential field 32 results in the tissue adjacent to the column B of the first DBS device 2. A nearly zero potential field results in the tissue adjacent to the columns C and A.

As shown by Fig. 2B, at distances of the order of several centimetres, a potential of 10 -20 millivolts (mV) may result when an excitation of 2 volts is applied, which is an order of 1-2 magnitude larger than brain-signals, i.e a very easy and accurate measurement is secured by the system.

While rotating the dipole field from the first DBS device 2, a time-varying potential will be sensed by the second DBS device 10. This can be achieved for example by feeding the electrodes of the first DBS device with time-dependent excitation profiles for each set of the electrodes belonging the various columns A;B;C;D as illustrated in Fig. 2C. By measuring a sensing phase of this sensed signal 8 which is shown in Fig. 2D with respect to the excitation profiles applied to the first DBS device 2, the orientation of the first DBS device with respect to the second DBS device is obtained. This is done by comparing the present phase of the sensing signal with the present phases of each of the time-varying signals. As the second DBS device 10 is positioned medially, i.e. on the other brain hemisphere, the relative position of the first DBS device in respect to the second DBS device is known. In this way the orientation of the first DBS device 2 with respect to patient's brain is unambiguously determined.

Such system 1 can be also used during a surgical implantation procedure.

Non-sinusoidal time-dependent excitation profiles can be also used, for example block-like excitation profiles.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the appended claims. In the claims, the word "comprising" does not exclude other elements or steps. Any reference signs in the claims should not be construed as limiting the scope.

### LIST OF REFERENCE NUMERALS:

- 1: a system
- 2: a first biomedical stimulation device
- 4A;4B;4C;4D: a stimulator element
- 6A,6B,6C,6D: a time-varying electric signal
- 8: a sensing signal
- 10: a sensor device
- 12: a generator
- 14: an orientation of the first biomedical stimulation device
- 20: an object
- 30: a negative potential field
- 32: a positive potential field

## Claims

1. A system (1) for determination of an orientation (14) of a first biomedical stimulation device (2) in respect to an object (20), the system comprising:
- the first biomedical stimulation device (2) comprising at least two stimulator elements (4A;4C),
- a generator (22) configured to simultaneously feed the stimulator elements (4A;4C) with time-varying electric signals (6A;6C),
- a sensor device (10) with a known relative position (X;α) with respect to the first biomedical stimulation device (2), arranged for sensing a sensing signal (8) from the stimulator elements (4A;4C), wherein the sensing signal (8) is originated from the time-varying electric signals (6A;6C) from the stimulator elements (4A;4C), and
- a processor device (12) configured to determine the orientation (14) of the first biomedical stimulation device (2) based on a sensed phase of the sensing signal (8), phases of the time-varying signals (6A;6C) and the known relative position (X;α),
**characterized in that** the time-varying electric signals (6A;6C) mutually differ by a predetermined phase.

2. The system (1) as claimed in claim 1, wherein the first biomedical stimulation device (2) is implantable in the object (20).

3. The system (1) as claimed in claim 1, wherein the first biomedical stimulation device (2) is a first deep brain stimulation (DBS) device (2).

4. The system (1) as claimed in claim 1, wherein the sensor device (10) is implantable in the object (20).

5. The system (1) as claimed in claim 1, wherein the sensor device (10) is a second biomedical stimulation device (10).

6. The system (1) as claimed in claim 5, wherein the second biomedical stimulation device (10) is a second deep brain stimulation (DBS) device (10).

7. The system (1) as claimed in claim 1, wherein the time-varying electric signals (6A;6C) are sinusoidal patterns.

8. A method for determination of an orientation (14) of a first biomedical stimulation device (2) in respect to an object (20), wherein the first biomedical stimulation device (2) comprises stimulator elements (4A;4C), the method comprising the following steps:
- feeding the stimulator elements (4A;4C) by a generator (12) with time-varying electric signals (6A;6C),
- sensing a sensing signal (8) from the stimulator elements (4A;4C) by a sensor device (10) with a known relative position (X;α) with respect to the first biomedical stimulation device (2), wherein the sensing signal (8) is originated from the time-varying electric signals (6A;6C) from the stimulator elements (4A;4C), and
- determining the orientation (14) of the first biomedical stimulation device (2) based on a sensed phase of the sensing signal (8), phases of the time-varying signals (6A;6C) and the known relative position (X;α) by a processor device (12),
**characterized in that** the time-varying electric signals (6A;6C) mutually differ by a predetermined phase.

## Patentansprüche

1. System (1) zur Bestimmung einer Ausrichtung (14) eines ersten biomedizinischen Stimulationsgeräts (2) in Bezug auf ein Objekt (20), wobei das System Folgendes umfasst:
- das erste biomedizinische Stimulationsgerät (2), das wenigstens zwei Stimulatorelemente (4A; 4C) umfasst,
- einen Generator (22), der so ausgebildet ist, dass er den Stimulatorelementen (4A; 4C) sich zeitlich ändernde elektrische Signale (6A; 6C) gleichzeitig zuführt,
- eine Sensorvorrichtung (10) mit einer bekannten relativen Position (X; α) bezüglich des ersten biomedizinischen Stimulationsgeräts (2), die so angeordnet ist, dass sie ein Abfühlsignal (8) von den Stimulatorelementen (4A; 4C) abfühlt, wobei das Abfühlsignal (8) von den sich zeitlich ändernden elektrischen Signalen (6A; 6C) von den Stimulatorelementen (4A; 4C) stammt, und
- eine Prozessorvorrichtung (12), die so ausgebildet ist, dass sie die Ausrichtung (14) des ersten biomedizinischen Stimulationsgeräts (2) anhand einer abgefühlten Phase des Abfühlsignals (8), von Phasen der sich zeitlich ändernden Signale (6A; 6C) und der bekannten relativen Position (X; α) bestimmt,
**dadurch gekennzeichnet, dass** die sich zeitlich ändernden elektrischen Signale (6A; 6C) um eine vorbestimmte Phase voneinander verschieden sind.

2. System (1) nach Anspruch 1, bei dem das erste biomedizinische Stimulationsgerät (2) in das Objekt (20) implantierbar ist.

3. System (1) nach Anspruch 1, bei dem das erste biomedizinische Stimulationsgerät (2) ein erstes Gerät (2) zur tiefen Hirnstimulation (DBS, deep brain stimulation) ist.

4. System (1) nach Anspruch 1, bei dem die Sensorvorrichtung (10) in das Objekt (20) implantierbar ist.

5. System (1) nach Anspruch 1, bei dem die Sensorvorrichtung (10) ein zweites biomedizinisches Stimulationsgerät (10) ist.

6. System (1) nach Anspruch 5, bei dem das zweite biomedizinische Stimulationsgerät (10) ein zweites Gerät (10) zur tiefen Hirnstimulation (DBS) ist.

7. System (1) nach Anspruch 1, bei dem die sich zeitlich ändernden elektrischen Signale (6A; 6C) Sinusmuster sind.

8. Verfahren zur Bestimmung einer Ausrichtung (14) eines ersten biomedizinischen Stimulationsgeräts (2) in Bezug auf ein Objekt (20), wobei das erste biomedizinische Stimulationsgerät (2) Stimulatorelemente (4A; 4C) umfasst, wobei das Verfahren die folgenden Schritte umfasst:
- den Stimulatorelementen (4A; 4C) werden von einem Generator (12) sich zeitlich ändernde elektrische Signale (6A; 6C) zugeführt,
- ein Abfühlsignal (8) von den Stimulatorelementen (4A; 4C) wird von einer Sensorvorrichtung (10) mit einer bekannten relativen Position (X; α) bezüglich des ersten biomedizinischen Stimulationsgeräts (2) abgefühlt, wobei das Abfühlsignal (8) von den sich zeitlich ändernden elektrischen Signalen (6A; 6C) von den Stimulatorelementen (4A; 4C) stammt, und
- die Ausrichtung (14) des ersten biomedizinischen Stimulationsgeräts (2) wird von einer Prozessorvorrichtung (12) anhand einer abgefühlten Phase des Abfühlsignals (8), von Phasen der sich zeitlich ändernden Signale (6A; 6C) und der bekannten relativen Position (X; α) bestimmt,
**dadurch gekennzeichnet, dass** die sich zeitlich ändernden elektrischen Signale (6A; 6C) um eine vorbestimmte Phase voneinander verschieden sind.

## Revendications

1. Système (1) pour la détermination d'une orientation (14) d'un premier dispositif de stimulation biomédical (2) par rapport à un objet (20), le système comportant :
- le premier dispositif de stimulation biomédical (2) qui présente au moins deux éléments de stimulateur (4A ; 4C),
- un générateur (22) réalisé de manière à fournir simultanément des signaux électriques variables dans le temps (6A ; 6C) aux éléments de stimulateur (4A ; 4C),
- un dispositif capteur (10) avec une position relative connue (X; α) par rapport au premier dispositif de stimulation biomédical (2) et qui est agencé de manière à détecter un signal détecteur (8) des éléments de stimulateur (4A ; 4C), le signal détecteur (8) provenant des signaux électriques variables dans le temps (6A ; 6C) des éléments de stimulateur (4A ; 4C), et
- un dispositif processeur (12) réalisé de manière à déterminer l'orientation (14) du premier dispositif de stimulation biomédical (2) sur la base d'une phase détectée du signal détecteur (8), de phases des signaux variables dans le temps (6A ; 6C) et de la position relative connue (X ; α),
**caractérisé en ce que** les signaux électriques variables dans le temps (6A ; 6C) sont différents les uns des autres d'une phase prédéterminée.

2. Système (1) selon la revendication 1, dans lequel le premier dispositif de stimulation biomédical (2) est apte à être implanté dans l'objet (20).

3. Système (1) selon la revendication 1, dans lequel le premier dispositif de stimulation biomédical (2) est un premier dispositif (2) de stimulation cérébrale profonde (DBS, deep brain stimulation).

4. Système (1) selon la revendication 1, dans lequel le dispositif capteur (10) est apte à être implanté dans l'objet (20).

5. Système (1) selon la revendication 1, dans lequel le dispositif capteur (10) est un deuxième dispositif de stimulation biomédical (10).

6. Système (1) selon la revendication 5, dans lequel le deuxième dispositif de stimulation biomédical (10) est un deuxième dispositif (10) de stimulation cérébrale profonde (DBS).

7. Système (1) selon la revendication 1, dans lequel les signaux électriques variables dans le temps (6A ; 6C) sont des formes sinusoïdales.

8. Procédé de détermination d'une orientation (14) d'un premier dispositif de stimulation biomédical (2) par rapport à un objet (20), dans lequel le premier dispositif de stimulation biomédical (2) comporte des éléments de stimulateur (4A ; 4C), le procédé comprenant les étapes suivantes :
- fournissement de signaux électriques variables dans le temps (6A; 6C) aux éléments de stimulateur (4A; 4C) au moyen d'un générateur (12),
- détection d'un signal détecteur (8) des éléments de stimulateur (4A ; 4C) par un dispositif capteur (10) avec une position relative connue (X ; α) par rapport au premier dispositif de stimulation biomédical (2), le signal détecteur (8) provenant des signaux électriques variables dans le temps (6A ; 6C) des éléments de stimulateur (4A ; 4C), et
- détermination de l'orientation (14) du premier dispositif de stimulation biomédical (2) sur la base d'une phase détectée du signal détecteur (8), de phases des signaux variables dans le temps (6A ; 6C) et de la position relative connue (X ; α) par un dispositif processeur (12),
**caractérisé en ce que** les signaux électriques variables dans le temps (6A ; 6C) sont différents les uns des autres d'une phase prédéterminée.
